# EUROPEAN PATENT APPLICATION

(11) **EP 4 131 116 A1**
(43) Date of publication of application: **08.02.2023**
(21) Application number: 20928924.8
(22) Date of filing: 25.12.2020
(51) Int. Cl.: G06Q 30/02, G06N 5/04, G06N 20/00

(54) **DESIGN EVALUATION DEVICE, LEARNING DEVICE, PROGRAM, AND DESIGN EVALUATION METHOD**

(30) Priority: 31.03.2020 JP 2020064557
(71) Applicant: Konica Minolta, Inc., Tokyo 100-7015 (JP)
(72) Inventor: INOUE, Satoru, Tokyo 100-7015 (JP); URATANI, Shoichi, Tokyo 100-7015 (JP); TERADA, Kenji, Tokyo 100-7015 (JP); SAITO, Shinichiro, Tokyo 100-7015 (JP); TAKASHIMA, Nobuhiko, Tokyo 100-7015 (JP)
(74) Representative: MFG Patentanwälte Meyer-Wildhagen Meggle-Freund Gerhard PartG mbB
(86) International application number: PCT/JP2020/048987
(87) International publication number: WO 2021/199541

(57) **Abstract**

A design evaluation device (100) includes a sensitivity information acquisition unit (111) that acquires sensitivity information of an evaluator with respect to a design of an object, an attribute information acquisition unit (112) that acquires attribute information of the evaluator, and a purchase expectation degree prediction unit (115) that predicts a purchase expectation degree with respect to the design of the object on the basis of the sensitivity information and the attribute information.

## Description

### Technical Field

The present invention relates to a design evaluation device, a learning device, a program, and a design evaluation method for evaluating a design in a product package, advertisement, or the like.

### Background Art

When a product is sold or advertised through various media such as magazines, televisions, and the Internet in addition to storefronts, a design of a product package and a design of an advertisement are major factors that affect the sales of the product. For this reason, the designs of the product package and the advertisement are important, and the product developer wants to employ a design that suits customer's preferences. According to an invention described in Patent Literature 1, it is possible to determine preferences of potential users with high accuracy.

### Citation List

### Patent Literature

Patent Literature 1: JP 2014-219937 A

### Summary of Invention

### Technical Problem

By using the invention described in Patent Literature 1, it is possible to determine the customer's preference for the design. However, even if a design having a high level of preference is employed for the product package or the advertisement, it does not necessarily lead to sales of the product. Even if the preference level of the customer is higher in a design B than in a design A, in a case where the customer is careful about consumption, the design B does not lead to purchase and does not lead to sales.

The present invention has been made in view of such a background, and an object thereof is to provide a design evaluation device, a learning device, a program, and a design evaluation method capable of evaluating a design including up to a possibility of purchase.

### Solution to Problem

The above object of the present invention is achieved by the following means.
(1) A design evaluation device, including a sensitivity information acquisition unit that acquires sensitivity information of an evaluator with respect to a design of an object, an attribute information acquisition unit that acquires attribute information of the evaluator, and a purchase expectation degree prediction unit that predicts a purchase expectation degree with respect to the design of the object on a basis of the sensitivity information and the attribute information.
(2) The design evaluation device according to (1), further including a share prediction unit that predicts a market share of the object by using the purchase expectation degree.
(3) The design evaluation device according to (1), further including a marketability determination unit that determines marketability by using the purchase expectation degree and additional information.
(4) The design evaluation device according to (1), in which a plurality of designs for the object is input, and the design evaluation device further includes a proposal unit that suggests an optimal design from among the plurality of designs using at least the purchase expectation degree.
(5) The design evaluation device according to (2), further including a purchase expectation degree correction value calculation unit that calculates a purchase expectation degree correction value for correcting the purchase expectation degree on a basis of the attribute information.
(6) The design evaluation device according to (1), in which the purchase expectation degree is a purchase probability.
(7) The design evaluation device according to (5), in which a market share in the share prediction unit is calculated using the purchase expectation degree that has been corrected with the purchase expectation degree correction value.
(8) The design evaluation device according to (5), in which the purchase expectation degree correction value calculation unit determines a similar type to which the evaluator belongs from the attribute information, and calculates a purchase expectation degree correction value allocated to the similar type as a purchase expectation degree correction value for correcting the purchase expectation degree.
(9) The design evaluation device according to (5), in which the purchase expectation degree correction value calculation unit determines a similar type to which the evaluator belongs from the attribute information, and calculates a purchase expectation degree correction value assigned to a combination of the similar type and a category of the object as a purchase expectation degree correction value for correcting the purchase expectation degree.
(10) The design evaluation device according to (1), in which the sensitivity information includes at least one of an answer of the evaluator to an inquiry about a favorable impression of the object, a time until the answer is made, an answer of the evaluator to an inquiry about a purchase intention of the object, a time until the answer is made, a time during which the evaluator gazes at the design of the object, or an electroencephalogram measurement result when the evaluator gazes at the design of the object.
(11) The design evaluation device according to (1), in which the sensitivity information acquired by the sensitivity information acquisition unit includes sensitivity information of the evaluator after a price of the object is presented.
(12) A learning device, including a learning data storage unit that stores, as learning data, a set of data of a design of an object, sensitivity information of an evaluator with respect to the design of the object, attribute information of the evaluator, and a purchase expectation degree of the evaluator for the object, a purchase behavior acquisition unit that acquires purchase behavior information of the evaluator of the object, and a purchase expectation degree learning unit that associates the purchase behavior information with learning data of the object, and generates a learned model of the purchase expectation degree with respect to the design.
(13) A design evaluation device, including a data acquisition unit that acquires data of a design of a second object, and a second object purchase expectation degree output unit that outputs a purchase expectation degree of the second object generated with the data and the learned model generated by the learning device according to (12).
(14) The design evaluation device according to (13), further including a share prediction unit that predicts a market share of the second object by using the purchase expectation degree.
(15) The design evaluation device according to (13) or (14), further including a marketability determination unit that determines marketability by using information of the purchase expectation degree and additional information.
(16) The design evaluation device according to any one of (13) to (15), in which a plurality of designs for an object is input, and the design evaluation device further includes a proposal unit that suggests an optimal design from among a plurality of objects using at least the purchase expectation degree.
(17) The design evaluation device according to any one of (13) to (16), further including an image feature calculation unit that calculates feature amounts of a design stored in a storage unit of the learning device and a design of the second object, and an extraction unit that compares the feature amount of the design stored in the storage unit of the learning device with the feature amount of the design of the second object, and extracts a design closest to the design of the second object from among designs in teacher data of the learning device.
(18) A program for causing a computer to function as the design evaluation device according to any one of (1) to (11) and (13) to (17).
(19) A design evaluation method executed by a design evaluation device, the method including a step of acquiring sensitivity information of an evaluator with respect to a design of an object, a step of acquiring attribute information of the evaluator, and a step of predicting a purchase expectation degree with respect to the design of the object on a basis of the sensitivity information and the attribute information.

### Advantageous Effects of Invention

According to the present invention, a design evaluation device, a learning device, a program, and a design evaluation method capable of evaluating a design including up to a possibility of purchase can be provided.

### Brief Description of Drawings

Fig. 1 is a functional block diagram of a design evaluation device according to a first embodiment.
Fig. 2 is a data configuration diagram of an evaluation database according to the first embodiment.
Fig. 3 is a classification example (1) according to the first embodiment.
Fig. 4 is a classification example (2) according to the first embodiment.
Fig. 5 is a data configuration diagram of a correction value database according to the first embodiment.
Fig. 6 is a flowchart illustrating a procedure of design evaluation according to the first embodiment.
Fig. 7 is a flowchart of design evaluation processing according to the first embodiment.
Fig. 8 is a functional block diagram of a design evaluation device according to a second embodiment.
Fig. 9 is a data configuration diagram of an evaluation database according to the second embodiment.
Fig. 10 is a flowchart of similar type estimation processing with a large purchase amount according to the second embodiment.
Fig. 11 is a functional block diagram of a design evaluation device and a learning device according to a third embodiment.
Fig. 12 is a flowchart of generation processing of a purchase expectation degree learning model according to the third embodiment.
Fig. 13 is a flowchart of correction value update processing according to the third embodiment.

### Description of Embodiments

### «Outline of Design Evaluation Device»

Next, a design evaluation device in a mode (embodiment) for carrying out the present invention will be described. A design evaluation device acquires an attribute of an evaluator who evaluates a design of a product package or a design of an advertisement or the like and sensitivity information of the evaluator with respect to the design. The evaluator is, for example, a consumer extracted from the market such as an applicant for a product test and is a potential purchaser of the product.

The sensitivity information is the design of the product package and the design of the advertisement, and further a favorable impression, a degree of desire to buy, and a degree of attention for the product. The design evaluation device calculates a degree to which the evaluator purchases the product (purchase probability and purchase expectation degree) from the sensitivity information. The attribute of the evaluator is a demographic attribute, a psychological attribute, and a behavioral attribute. The design evaluation device corrects the purchase probability of the evaluator using a similar type determined from the attribute of the evaluator. For example, in a case where the evaluator belongs to the similar type that is considered to be careful or passive, the design evaluation device corrects the purchase probability to be low. The design evaluation device predicts a market share and a purchase amount (sales) of a product from the corrected purchase probability.

### «Configuration of Design Evaluation Device»

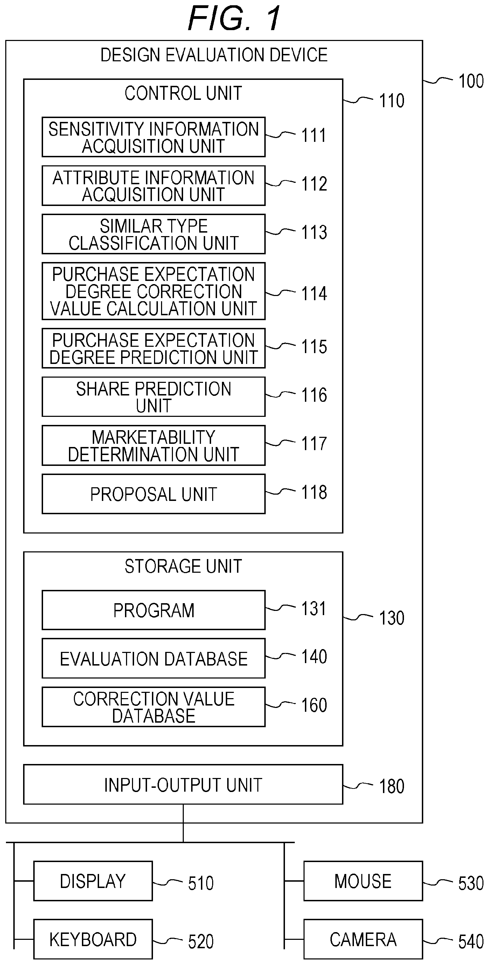

Fig. 1 is a functional block diagram of a design evaluation device 100 according to a first embodiment. The design evaluation device 100 includes a control unit 110, a storage unit 130, and an input-output unit 180. A user interface device, such as a display 510, a keyboard 520, and a mouse 530, is connected to the input-output unit 180. In addition, to the input-output unit 180, a camera 540 for acquiring feeling information of the evaluator may be connected, or a device for measuring biological information of the evaluator, such as pulses, skin potential, and brain waves, may be connected. Results calculated, estimated, predicted, or determined by the functional units of the control unit 110 are output (displayed) to the display 510.

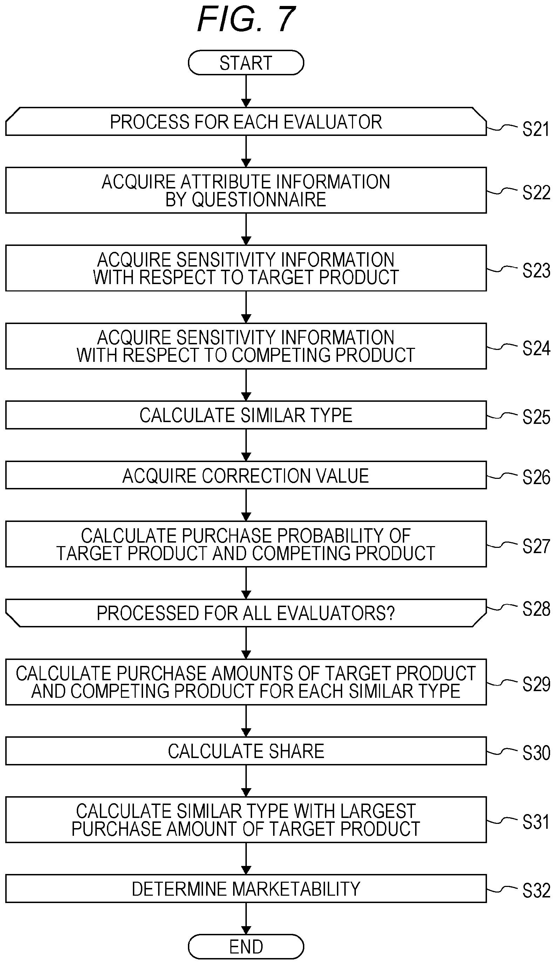

The storage unit 130 includes a storage device such as a read only memory (ROM), a random access memory (RAM), or a solid state drive (SSD). The storage unit 130 stores a program 131, an evaluation database 140, and a correction value database 160. The program 131 is a program executed by the control unit 110 to be described later, and includes a procedure of design evaluation processing (see Fig. 7 to be described later).

### «Configuration of Design Evaluation Device: Evaluation Database»

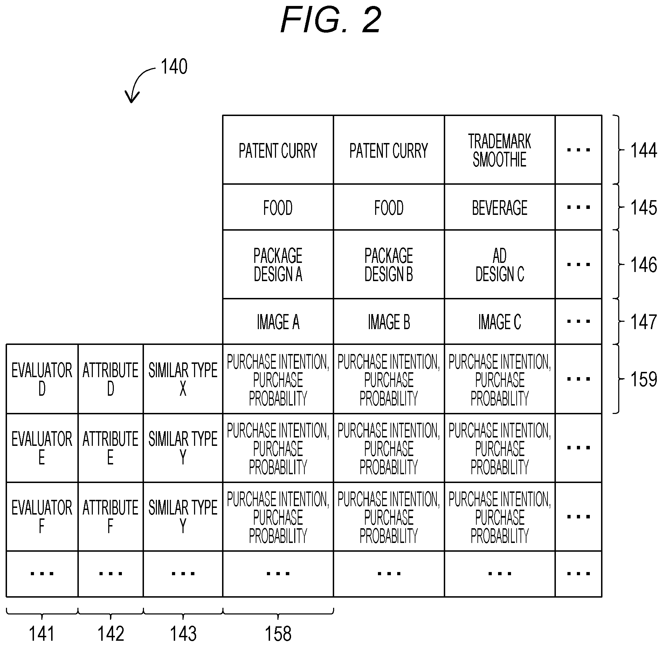

Fig. 2 is a data configuration diagram of the evaluation database 140 according to the first embodiment. The evaluation database 140 includes an evaluation of the evaluator for the design. A row of the evaluation database 140 is a record of the evaluator including information (item) related to the evaluator and an evaluation of the design by the evaluator. A column of the evaluation database 140 is a record of a design including information (item) related to the design and an evaluation of the design by the evaluator.

The record of the evaluator includes identification information 141, an attribute 142, a similar type 143, and an evaluation for each design.

The identification information 141 is identification information of the evaluator.

The attribute 142 is an attribute of the evaluator. The attributes of the evaluator are classified into three types of demographic attribute, psychological attribute, and behavioral attribute. The demographic attribute is an attribute such as age (year), sex, and residential place. The psychological attribute is an attribute such as a value and a consumption propensity. The behavioral attribute is an attribute such as a product purchase history and a behavior range.

For example, the demographic attribute can be acquired by asking age or the like. Further, the psychological attribute can be acquired by asking whether to buy products frequently bought by other people, whether to buy brand products, and the like. Furthermore, it is possible to acquire the behavioral attribute by asking the evaluator about what he or she has bought recently, how to frequently purchase products, or the like in a questionnaire, or by acquiring the history of the web.

It is also possible to acquire the attribute by analyzing the behavior (action) of the evaluator instead of the questionnaire. For example, it is also possible to estimate the age from a face image and to determine the personality or the like from a behavior or movement of the line of sight. For example, a purchase behavior of the evaluator is captured by a camera in the store, and an evaluator who views a description on a product package for a long time before purchase can be determined to have a careful or logical personality. Further, when the questionnaire and behavior analysis are used together, it is possible to make a determination (acquire attributes) with high accuracy.

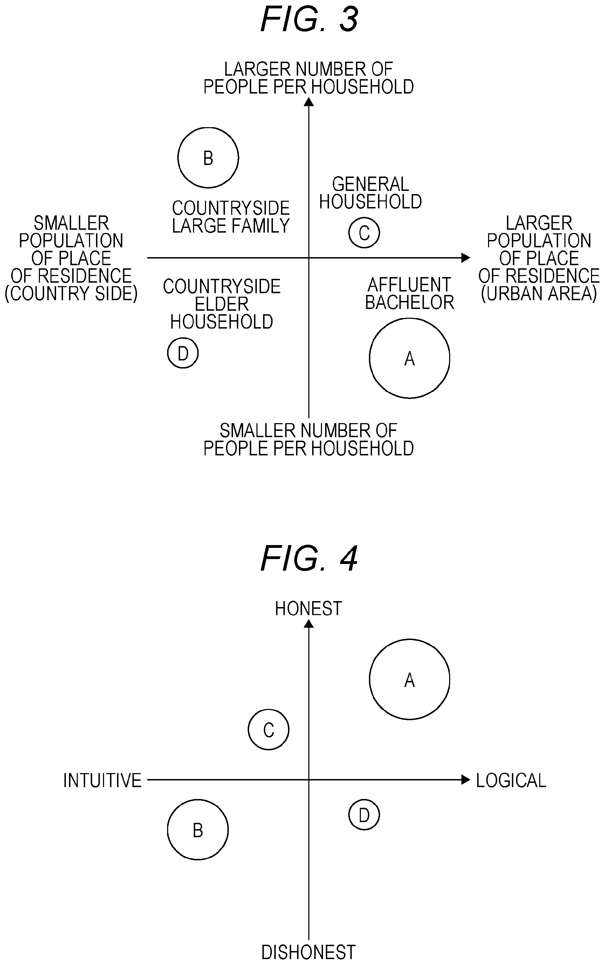

The similar type 143 is a similar type (see Figs. 3 and 4 described below) of results obtained by classifying the evaluator on the basis of the attribute 142 of the evaluator.

Fig. 3 is a classification example (1) according to the first embodiment. In Fig. 3, evaluators are classified according to the number of people per household and the place of residence. The size of a circle indicating a similar type indicates a population of similar types. For example, an evaluator having a large residence population and a small number of people per household is classified into a similar type of an affluent bachelor type. Further, the population of affluent bachelors is larger than that of similar types of other countryside large families and general households.

Fig. 4 is a classification example (2) according to the first embodiment. In Fig. 4, the evaluators are classified on two axes, namely, honest or dishonest and logical or intuitive.

Returning to Fig. 2, the value of the similar type 143 is not limited to one, and a plurality of similar types may be included by a classification method as illustrated in Figs. 3 and 4. For classification into the similar type, for example, a clustering (cluster analysis) method may be used, or another method may be used.

The evaluation will be described later.

Following the record of the evaluator, the record of the design will be described. The record of the design includes a product name 144, a product classification 145, a design 146, an image 147, and an evaluation by each evaluator.

The product name 144 is a name of a product related to design.

The product classification 145 is a classification (category) of a product related to design. Examples of the classification of products include food, beverage, clothing, and the like.

The design 146 is identification information given to a design of a product package or a design of an advertisement.

The image 147 is an image of the design 146.

The item at which the record of the evaluator and the record of the design intersect is the evaluation of the design by the evaluator. For example, the evaluation at which the record 159 of the evaluator and the record 158 of the design intersect is the evaluation of the "package design A" by the "evaluator D".

The evaluation is an evaluation for the design by an evaluator, and is a purchase expectation degree. The purchase expectation degree includes a purchase intention and a purchase probability. The purchase intention indicates whether or not the evaluator who has viewed the product package or the advertisement has indicated an intention to purchase the product, and a degree of intention to purchase the product. The purchase intention may be acquired by asking the evaluator, or may be acquired by estimating the degree of attention (such as time for gazing at the design) from movement of the line of sight. The purchase intention may be a numerical value indicating the height, or may be a level such as high, medium, or low. In addition, a difference in purchase intention between the current design and a new design or the like may be expressed.

The purchase probability is a probability of purchasing a product. If the purchase intention is high, the purchase probability is expected to be high. However, even if the purchase intention is the same numerical value or level, the probability of purchasing differs depending on the evaluator. For example, it is considered that an evaluator with a large disposable income or an evaluator without a supporter actually purchases a product in many cases (probability is high). In addition, it is considered that the evaluator who likes new things or the evaluator who is impatient rather than the evaluator who is careful often purchases (probability is high). Such a coefficient for correcting the purchase intention of the evaluator for the product to the purchase probability is a correction value (also referred to as a purchase expectation degree correction value).

In the first embodiment, it is assumed that the purchase probability is determined not only by the purchase intention but also by various attributes of the evaluator and the product classification 145. Furthermore, the classification of the evaluator into the similar type 143 is performed so that the correction values are close among the similar types. Thus, the correction value is determined by the similar type 143 and the product classification 145 of the evaluator.

### «Configuration of Design Evaluation Device: Correction Value Database»

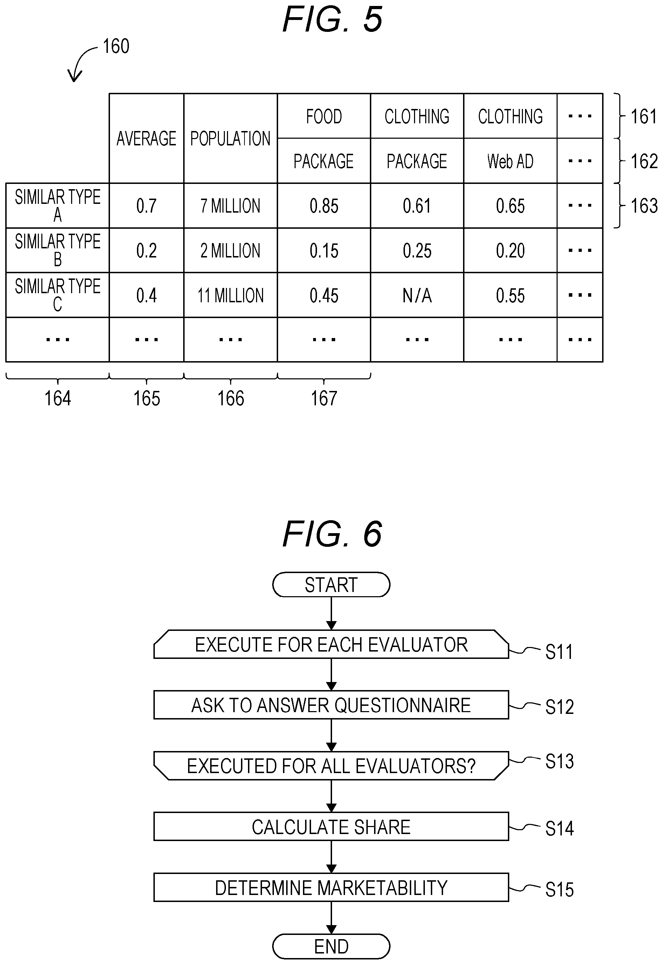

Fig. 5 is a data configuration diagram of the correction value database 160 according to the first embodiment. The correction value database 160 stores correction values corresponding to the similar type of the evaluator, the product classification, and the medium. A row of the correction value database 160 is a record of the similar type including the correction value of the design by the similar type. A column of the correction value database 160 is a record of the design including correction values of similar types for the design.

The record of the similar type includes a similar type 164, an average 165, a population 166, and a correction value for each design.

The similar type 164 is a similar type of evaluators and corresponds to the similar type 143 (see Fig. 2) of the evaluation database 140.

The average 165 is an average of the correction values in the similar types.

The population 166 is a population of the similar types.

The record of the design includes a product classification 161, a medium 162, and the correction value of each similar type. The product classification 161 is a classification of a product and corresponds to the product classification 145 (see Fig. 2). The medium is a design medium, and examples thereof include a "package" which is a package design, a "web ad" which is an advertisement on the web, and the like.

The item at which the record of the similar type and the record of the design intersect is the correction value of the design by the similar type. For example, the item at which the record 163 of the similar type and the record 167 of the design intersect is the correction value of a package design of food by the "similar type A", and the value thereof is "0.85".

### «Configuration of Design Evaluation Device: Control Unit»

Returning to Fig. 1, the description of the control unit 110 will be continued. The control unit 110 includes a central processing unit (CPU). The CPU executes the program 131 to perform design evaluation processing (see Fig. 7 described later). The control unit 110 includes a sensitivity information acquisition unit 111, an attribute information acquisition unit 112, a similar type classification unit 113, a purchase expectation degree correction value calculation unit 114, a purchase expectation degree prediction unit 115, a share prediction unit 116, a marketability determination unit 117, and a proposal unit 118.

The sensitivity information acquisition unit 111 acquires the sensitivity information of the evaluator. The sensitivity information is the purchase intention for the design. For example, the sensitivity information acquisition unit 111 displays the design on the display 510 (see Fig. 1), and acquires the level of the purchase intention selected (answered by the evaluator) from choices by the evaluator. The design viewed by the evaluator may be an actual product package or an advertisement poster instead of the design displayed on the display 510. The purchase intention of the answer may be changed according to an answer time of the evaluator. In addition, the sensitivity information acquisition unit 111 may acquire the purchase intention by showing the price of the product to the evaluator, may acquire the purchase intention without showing the price, or may acquire the purchase intention before and after showing the price.

In addition, the sensitivity information acquisition unit 111 may track the gaze of the evaluator to the design acquired by the camera 540, and estimate the purchase intention from the attention time. In addition, the sensitivity information acquisition unit 111 may estimate the purchase intention from a measurement result of biological information such as pulses, skin potential, and brain waves of the evaluator. The acquired purchase intention is stored in the evaluation database 140 (see Fig. 2) as a purchase intention of an evaluation item corresponding to the evaluator and the design.

The attribute information acquisition unit 112 acquires the attribute of the evaluator. For example, the attribute information acquisition unit 112 may display a question related to the attribute on the display 510, acquire an answer of the evaluator, and acquire the attribute. The acquired attribute is stored in the evaluation database 140 (see Fig. 2) as the attribute 142 of the evaluator. The attribute information acquisition unit 112 may acquire answers of the evaluator in the questionnaire form using, for example, an optical character recognition (OCR) technology.

The similar type classification unit 113 calculates the similar type to which the evaluator belongs from the attribute 142 and stores the same in the similar type 143 of the evaluation database 140.

The purchase expectation degree correction value calculation unit 114 acquires the corresponding correction value from the similar type 164 (see Fig. 5) of the evaluator, the product classification 161, and the medium 162.

The purchase expectation degree prediction unit 115 calculates the purchase expectation degree (including the purchase probability) from the correction value and the purchase intention.

The share prediction unit 116 calculates the purchase probability for each of the similar type to predict the purchase amount of the product, and predicts the market share from the purchase amount of the product.

The marketability determination unit 117 acquires additional information such as cost of the product and a competitive analysis such as a 3C analysis, and determines and outputs the marketability of the product. For example, in a case where the number of competing products is larger than a predetermined number, the market share predicted by the share prediction unit 116 is smaller than a predetermined value, or the purchase probability (average purchase probability) is lower than the predetermined value, the marketability determination unit 117 determines that the market is low and outputs this determination. In addition, in a case where the purchase intention after showing the price of the product is equal to or less than a predetermined ratio of the purchase intention before showing the price, the marketability determination unit 117 may determine that the marketability is low. If the difference from the purchase probability of the competing product is equal to or more than the predetermined value, the marketability determination unit 117 may determine that the marketability is high.

The proposal unit 118 searches for and outputs the similar type with a largest purchase amount of the product calculated from the purchase probability.

### <<Design Evaluation Procedure>>

Fig. 6 is a flowchart illustrating a procedure of design evaluation according to the first embodiment.

In steps S11 to S13, each evaluator answers the questionnaire. Specifically, each evaluator answers a question about the design of the product (question about the purchase intention) or a question about the evaluator (question about the attribute of the evaluator) displayed on the display 510 of the design evaluation device 100. Note that the product is not limited to the product as a design evaluation target, and includes competing products required for calculating the market share.

In step S14, the design evaluation device 100 calculates and outputs the market share of each product.

In step S15, the design evaluation device 100 acquires the input additional information (information regarding product costs, competing products, 3C analysis, and the like), and determines and outputs the marketability of the product.

### <<Design Evaluation Processing>>

Fig. 7 is a flowchart of design evaluation processing according to the first embodiment.

In step S21, the control unit 110 starts processing of repeating the processing in steps S22 to S27 for each evaluator.

In step S22, the attribute information acquisition unit 112 acquires the attribute information of the evaluator according to the questionnaire. Specifically, the attribute information acquisition unit 112 displays a question item to the evaluator on the display 510 (see Fig. 1) and acquires an answer from the evaluator. The attribute information acquisition unit 112 stores the attribute obtained from the answer in the attribute 142 of the evaluation database 140 (see Fig. 2).

In step S23, the sensitivity information acquisition unit 111 displays the design of the product (product package design or advertisement design) as the design evaluation target on the display 510, and acquires the purchase intention as the sensitivity information. Specifically, the sensitivity information acquisition unit 111 displays the design on the display 510, and acquires the level of the purchase intention selected from choices by the evaluator. The sensitivity information acquisition unit 111 stores the acquired purchase intention in the evaluation item of the evaluation database 140.

In step S24, the sensitivity information acquisition unit 111 displays the design of the competing product on the display 510, and acquires the purchase intention as the sensitivity information. The sensitivity information acquisition unit 111 stores the acquired purchase intention in the evaluation of the evaluation database 140. The number of competing products is not limited to one, and there may be a plurality of competing products.

In step S25, the similar type classification unit 113 calculates the similar type from the attribute 142 of the evaluator and stores the same in the similar type 143.

In step S26, the purchase expectation degree correction value calculation unit 114 specifies the similar type 164, the product classification 161, and the medium 162 of the correction value database 160 (see Fig. 5) corresponding to the similar type 143 of the evaluator, the product classification 145, and the design 146 (see Fig. 2), and acquires the corresponding correction value. Note that, in a case where there are no product classification 161 and no medium 162 corresponding to the product classification 145 and the design 146, the purchase expectation degree correction value calculation unit 114 sets the average 165 as the correction value.

In step S27, the purchase expectation degree prediction unit 115 calculates the purchase probability from the correction value and the purchase intention acquired in step S26, and stores the purchase probability in the evaluation item. The purchase expectation degree prediction unit 115 calculates a purchase probability for each of the evaluation target product and the competing product. When the purchase intention is a level, the level is converted into a numerical value of zero to one and multiplied by the correction value to calculate the purchase probability.

In step S28, the control unit 110 proceeds to step S29 if the processing in steps S22 to S27 has been completed for all evaluators, and returns to step S22 if there is an evaluator for whom the processing has not been completed (who has not answered the questionnaire).

In step S29, the share prediction unit 116 calculates the purchase amounts of the product as the design evaluation target and the competing product. Specifically, the share prediction unit 116 calculates an average of the purchase probabilities of the product as the evaluation target of the evaluation target and the competing product belonging to the similar type for each of the similar type. Next, the share prediction unit 116 multiplies the purchase probability by the population 166 to obtain the purchase amount of each of the product as the evaluation target of the evaluation target and the competing product.

In step S30, the share prediction unit 116 calculates and outputs the market share. Specifically, the share prediction unit 116 calculates a ratio of each of the evaluation target product and the competing product with the sum of the purchase amounts of the evaluation target product and the competing product being the total purchase amount, and outputs the ratio as the market share.

In step S31, the proposal unit 118 outputs the similar type with the largest purchase amount of the evaluation target product as a similar type with the largest purchase. The similar type with largest purchase is used as a sales promotion target.

In step S32, the marketability determination unit 117 acquires the additional information such as the cost of the product and a competition analysis such as a 3C analysis, and determines and outputs the marketability of the product.

### <<Characteristics of Design Evaluation Processing>>

The design evaluation device 100 calculates the purchase probability in consideration of the correction value given to the similar type determined by the attribute of the evaluator in addition to the purchase intention of the evaluator for the product. Furthermore, the design evaluation device 100 calculates the purchase amount by multiplying the purchase probabilities of the product and the competing product calculated for each of the similar type by the population of the similar type, and calculates and estimates the market share.

The purchase probability is calculated in consideration of not only the intension and sensibility of the evaluator (customer or purchaser) who simply wants to purchase/does not purchase or likes/dislikes the product but also the similar type of the evaluator. The purchase probability can be estimated with high accuracy and the market share of the product can be calculated with high accuracy by classifying the product into the similar type and assigning the correction value to the similar type in consideration of the attribute of the purchaser that affects the purchase of the product other than the product itself and the design, such as the amount of disposable income, the number of dependent family members, and whether or not the purchaser likes new products.

Further, the design evaluation device 100 determines and outputs the marketability from the additional information.

### <<Modification Example of First Embodiment: Case Where There Is Plurality of Designs>>

In the above-described embodiment, the number of designs as the evaluation target has been described as one, but the number is not limited thereto. A plurality of designs may be input for one product and registered in the evaluation database 140, and the sensitivity information acquisition unit 111 may acquire the purchase intention of the evaluator for each design. Furthermore, the share prediction unit 116 may calculate the purchase probability for each design and predict the purchase amount. The proposal unit 118 may output and propose the design with the largest purchase amount as an optimal design for the product.

### «Modification Example of First Embodiment: Case Where Current Design and New Design Are Present»

The sensitivity information acquisition unit 111 acquires the purchase intention of the evaluator for the design. In a case where there is a design of a current product, instead of acquiring a purchase intention a new design, the sensitivity information acquisition unit 111 may acquire whether or not the purchase intention by the evaluator who has viewed both the current design and the new design has increased, and may assume this as the purchase intention. In addition, whether or not the purchase intention after showing the price has increased from the purchase intention before showing the price of the product may be acquired and assumed as the purchase intention. Note that it is also referred to as a purchase expectation degree as a superordinate concept including the purchase intention and the purchase probability acquired by the sensitivity information acquisition unit 111.

### <<Second Embodiment: Estimation of Purchase Intention by Design>>

In the first embodiment described above, the design evaluation device 100 shows the package design and the advertisement design of the product to the evaluator and acquires the purchase intention. The purchase intention may be estimated from a design similar to the design as the evaluation target. Furthermore, the similar type (sales promotion target) with the largest purchase amount may be output.

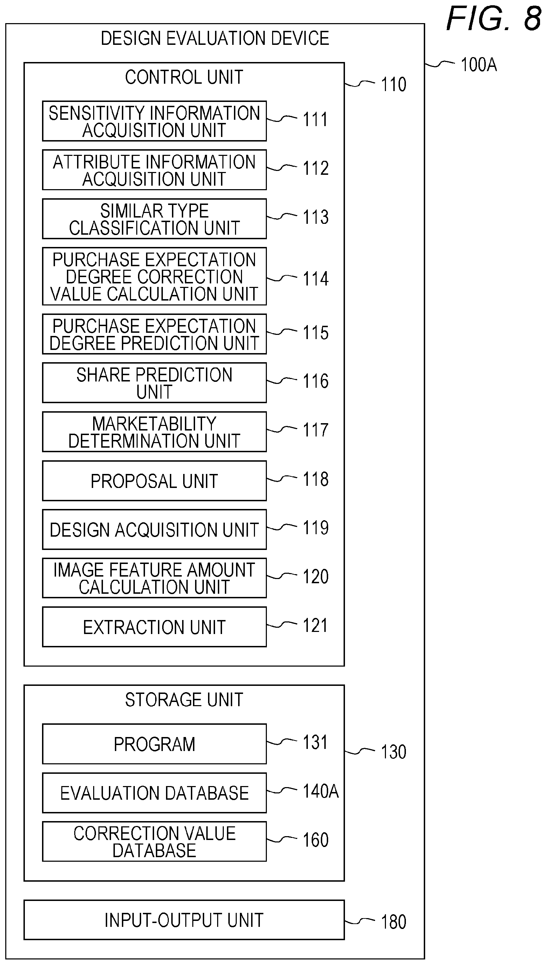

Fig. 8 is a functional block diagram of the design evaluation device 100A according to a second embodiment. An evaluation database 140A is different from the design evaluation device 100 of the first embodiment. Further, the control unit 110 includes a design acquisition unit 119, an image feature amount calculation unit 120, and an extraction unit 121.

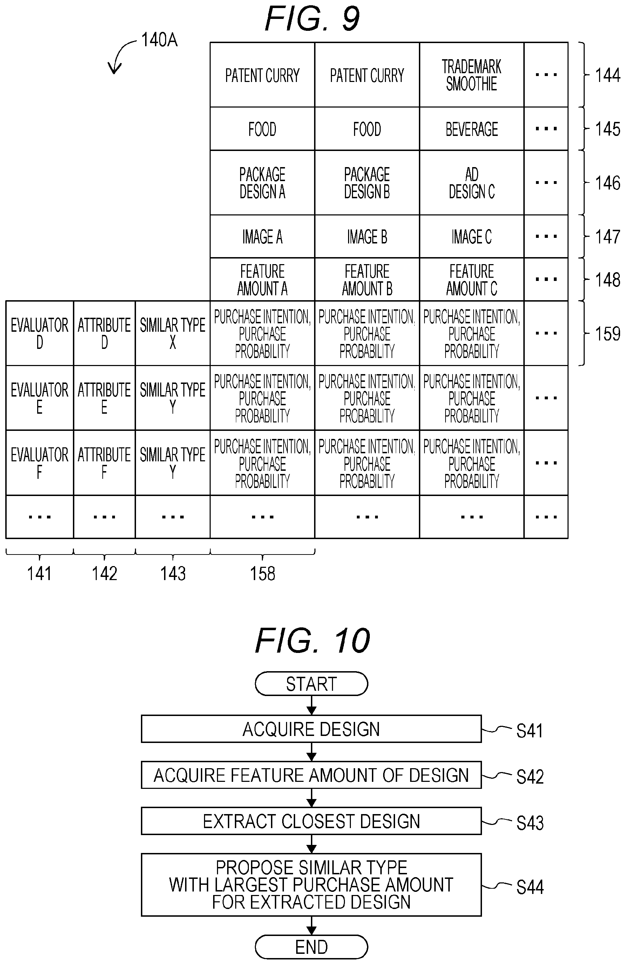

Fig. 9 is a data configuration diagram of the evaluation database 140A according to the second embodiment. As compared with the evaluation database 140 according to the first embodiment, the record (column) of the design includes a feature amount 148. The feature amount 148 is a feature amount of the image 147, and is, for example, a feature amount related to a gloss amount, a color tone, a color arrangement, presence/absence or arrangement of a characteristic figure/pattern, a character and a size, font, arrangement thereof, and the like.

Returning to Fig. 8, the design acquisition unit 119 acquires the design as the evaluation target.

The image feature amount calculation unit 120 calculates the feature amount from an image of the design as the evaluation target.

The extraction unit 121 extracts (specifies) a design similar to the design as the evaluation target from the evaluation database 140A. The extraction unit 121 specifies a design having the feature amount 148 closest to the feature of the image that is the design as the evaluation target.

### <<Second Embodiment: Estimation of Similar Type with Largest Purchase Amount>>

Fig. 10 is a flowchart of similar type estimation processing with a large purchase amount according to the second embodiment.

In step S41, the design acquisition unit 119 acquires a package of a product and a design of an advertisement as the evaluation target. For example, the design acquisition unit 119 acquires the design from the camera 540 (see Fig. 1).

In step S42, the image feature amount calculation unit 120 calculates the feature amount of the image having the design acquired in step S41.

In step S43, the extraction unit 121 extracts a record of a design having the feature amount 148 closest to the feature amount calculated in step S42 from the evaluation database 140A.

In step S44, the proposal unit 118 specifies and outputs the similar type with the largest purchase amount for the design extracted in step S43. The specifying method is similar to that of the first embodiment, and the proposal unit 118 specifies a similar type having the largest purchase amount from a result obtained by the share prediction unit 116 calculating the average value of the purchase probabilities for each similar type and multiplying the average value by the population 166 (see Fig. 5) to calculate the purchase amount.

### «Characteristics of Second Embodiment»

In the second embodiment, the design evaluation device 100 estimates the similar type with a largest purchase amount from a design in the evaluation database 140A that is most similar to the design as the evaluation target without acquiring the purchase intention of the evaluator for the design. Since evaluation by the evaluator is unnecessary, it is possible to reduce time and cost for gathering evaluators, acquiring a questionnaire, and the like.

### «Modification Example of Second Embodiment»

Note that, in the above-described embodiment, the extraction unit 121 focuses on the design acquired in step S41 and specifies a design having the most similar feature amount 148 among the designs of the evaluation database 140A. The extraction unit 121 may specify a design having the same product classification 145 and the most similar feature amount 148.

In addition, in the above-described embodiment, the design evaluation device estimates the similar type with the largest purchase amount from the most similar design, but the market share may be calculated or the marketability may be determined as in the first embodiment. Specifically, the design evaluation device may output the market share and the marketability related to the most similar design as the market share and the marketability of the design as the evaluation target.

Instead of specifying a design having a close design feature amount, a machine learning technique may be used. Specifically, a learning unit provided in the design evaluation device may generate a machine learning model using learning data to which a design (an image of a design or a feature amount of the image) is input and in which the similar type having the largest purchase amount is set as a correct answer label. Next, an estimation unit provided in the design evaluation device may estimate the similar type with the largest purchase amount from the design using the machine learning model.

The learning unit may estimate the market share and the marketability by generating the machine learning model using the market share and the marketability as correct answer labels, in addition to the similar type with the largest purchase amount. In addition, the estimation unit may estimate the market share and the marketability for each of the plurality of designs and output a design having high market share and marketability.

### <<Third embodiment: Learning Device, Learning of Purchase Expectation Degree, and Update of Correction Value>>

The purchase expectation degree including the purchase probability is determined by the sensitivity information of the evaluator and various attributes of the evaluator. The correction value is stored in the correction value database 160 (see Fig. 5), and is allocated for each similar type 164, each product classification 161, and each medium 162. A method of calculating a purchase expectation degree prediction value based on the sensitivity information or the attribute information may be learned on the basis of information obtained by checking with the evaluator whether or not the product has been actually purchased. In addition, the already-allocated correction value may be updated.

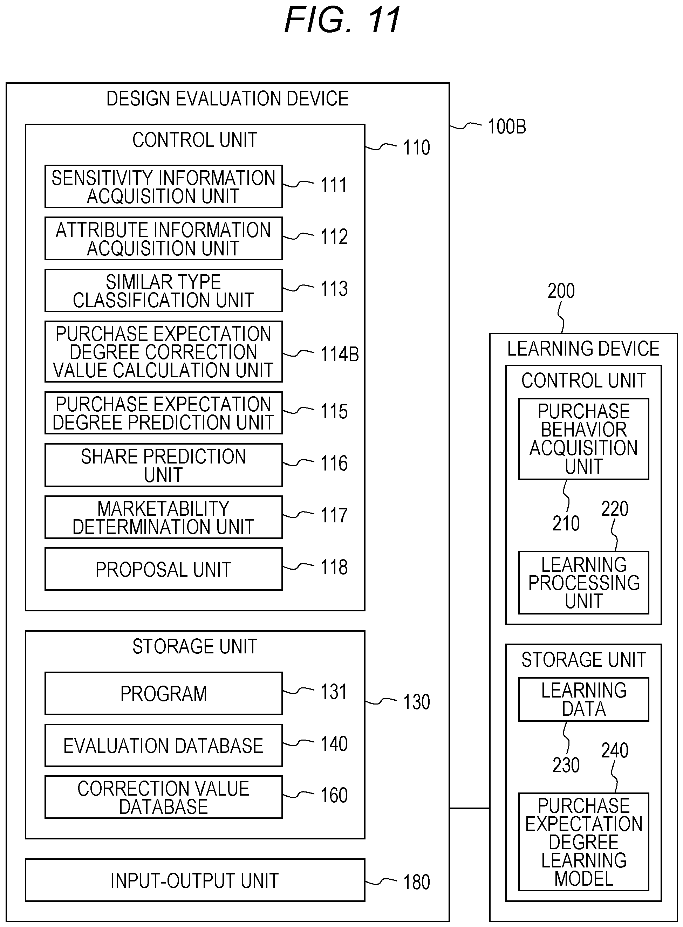

Fig. 11 is a functional block diagram of a design evaluation device 100B and a learning device 200 according to a third embodiment. Comparing the design evaluation device 100 according to the first embodiment with the design evaluation device 100B according to the third embodiment, a purchase expectation degree correction value calculation unit 114B is different. Further, a learning device 200 is connected to the design evaluation device 100B.

The learning device 200 includes a control unit including a CPU and a storage unit including a RAM, a ROM, an SSD, and the like. The storage unit (learning data storage unit) stores learning data 230 and a purchase expectation degree learning model 240. The purchase expectation degree learning model 240 is a machine learning model that calculates the purchase expectation degree from the design of an object, the sensitivity information of the evaluator with respect to the design, and the attribute information of the evaluator.

The learning data 230 is learning data for generating (training) the purchase expectation degree learning model 240, and is data including the design of an object, the sensitivity information of the evaluator with respect to the design, and the attribute information of the evaluator. The design of the object, the sensitivity information of the evaluator for the design, and the attribute information of the evaluator are acquired from the evaluation database 140 (see Fig. 2). The design is, for example, the image 147 of the design or the feature amount 148 thereof (see Fig. 9).

The control unit includes a purchase behavior acquisition unit 210 and a learning processing unit 220 (purchase expectation degree learning unit). The purchase behavior acquisition unit 210 acquires the presence or absence of purchase of product (purchase behavior information) from the evaluator. The learning processing unit 220 accumulates the learning data 230, and generates the purchase expectation degree learning model 240 using the learning data 230 and the purchase behavior information (trains the purchase expectation degree learning model 240 using the learning data 230 and the purchase behavior information).

The learning device 200 acquires the presence or absence of the purchase of product from the evaluator from the purchase behavior acquisition unit 210 in the learning data 230 including data acquired from the evaluation database 140, and generates the purchase expectation degree learning model 240 that calculates the purchase expectation degree from the design, the sensitivity information, and the attribute information. Further, the purchase expectation degree correction value calculation unit 114B acquires the presence or absence of the purchase of product of the evaluator from the purchase behavior acquisition unit 210, and updates the correction value.

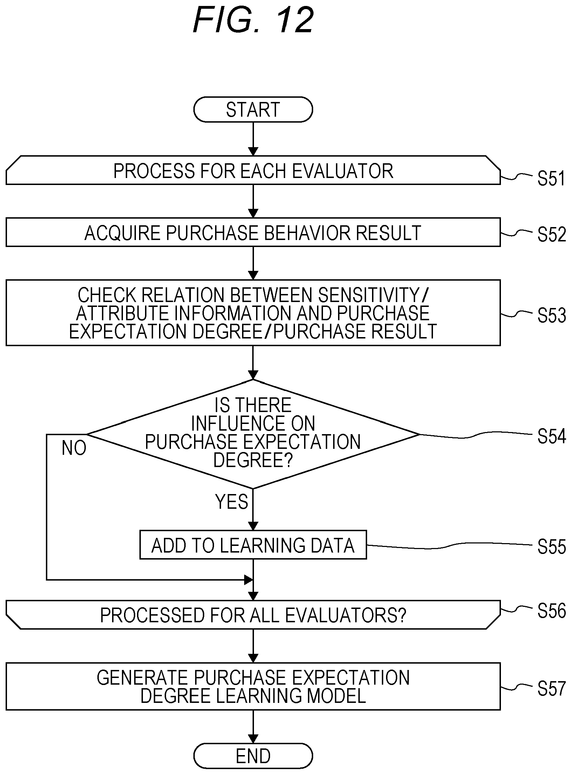

Fig. 12 is a flowchart of generation processing of the purchase expectation degree learning model 240 according to the third embodiment.

In step S51, the learning processing unit 220 starts processing of repeating steps S52 to S55 for each evaluator. The number of evaluators is, for example, 100.

In step S52, the purchase behavior acquisition unit 210 inquires of the evaluator and acquires a result of a purchase behavior (presence or absence of purchase).

In step S53, the learning processing unit 220 checks a relation between the sensitivity information and the attribute information of the evaluator and the purchase expectation degree and the purchase result.

In step S54, as a result of checking the relation in step S53, if there is an influence on the calculation of the purchase expectation degree (step S54 → YES), the learning processing unit 220 proceeds to step S55, and if there is no influence (step S54 → NO), the learning processing unit proceeds to step S56.

In step S55, the learning processing unit 220 adds data including a design of the object, the sensitivity information of the evaluator with respect to the design, and the attribute information to the learning data 230, and stores the data in association with the presence or absence of purchase, which is the correct answer label.

In step S56, the learning processing unit 220 proceeds to step S57 if steps S52 to S55 have been executed for all the evaluators, and returns to step S52 if there is an unprocessed evaluator.

In step S57, the learning processing unit 220 generates the purchase expectation degree learning model 240 on the basis of the learning data 230 and the presence or absence of the purchase of an evaluation.

By calculating the purchase expectation degree of the design evaluation device using the purchase expectation degree learning model 240 generated by the learning processing unit 220, it is possible to calculate an expectation degree closer to a real one.

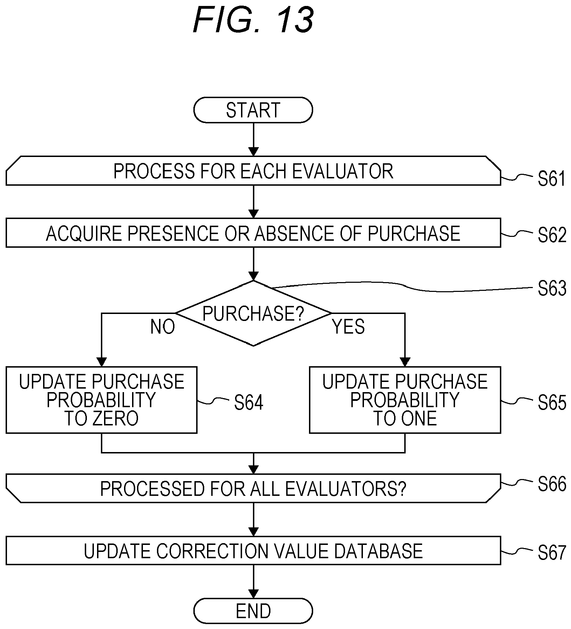

Fig. 13 is a flowchart of correction value update processing according to the third embodiment.

In step S61, the purchase expectation degree correction value calculation unit 114B starts processing of repeating steps S62 to S65 for each evaluator.

In step S62, the purchase expectation degree correction value calculation unit 114B acquires the presence or absence of the purchase of product from the purchase behavior acquisition unit 210 of the learning device 200.

In step S63, the purchase expectation degree correction value calculation unit 114B proceeds to step S65 if the evaluator has purchased the product (step S63 → YES), and proceeds to step S64 if the evaluator has not purchased the product (step S63 → NO).

In step S64, the purchase expectation degree correction value calculation unit 114B updates the purchase probability of the evaluation corresponding to the evaluator in the evaluation database 140 to zero.

In step S65, the purchase expectation degree correction value calculation unit 114B updates the purchase probability of the evaluation corresponding to the evaluator in the evaluation database 140 to one.

In step S66, if the purchase expectation degree correction value calculation unit 114B has executed steps S62 to S65 for all the evaluators, the process proceeds to step S67, and if there is an unprocessed evaluator, the process returns to step S62.

In step S67, the purchase expectation degree correction value calculation unit 114B updates the correction value database 160. Specifically, the purchase expectation degree correction value calculation unit 114B calculates the ratio of the evaluator who has purchased the product for each similar type, and calculates the correction value so that the ratio becomes equal to the purchase probability calculated from the purchase intention and the correction value.

The purchase expectation degree correction value calculation unit 114B may calculate the correction value according to the ratio of evaluators for whom the presence or absence of the purchase of product has been acquired. For example, (ratio of evaluators for whom the presence or absence of the purchase of product has been acquired) × (correction value by which the ratio of evaluators who have purchased the product becomes equal to the purchase probability calculated from the purchase intention and the correction value) + (ratio of evaluators for whom the presence or absence of the purchase of product has not been acquired) × (correction value before update) may be used as an update value.

### <<Characteristics of Third Embodiment>>

In the third embodiment, the design evaluation device 100B calculates an actual purchase probability by checking whether or not the evaluator has actually purchased the product, and updates the correction value so as to match the purchase probability. Updating the correction value in accordance with actual data improves the accuracy of subsequent prediction of the market share and estimation of the similar type with largest purchase.

### <<Modification Example of Third Embodiment>>

Note that, the learning device 200 has been mainly described in the above-described embodiment, as a design evaluation device using the purchase expectation degree learning model 240 generated by the learning device, it is possible to acquire a design of a second object as in the second embodiment, and then, for example, calculate the purchase expectation degree of the design of the second object from an image of the design or a design having a close feature amount of an image. In addition, the design evaluation device may extract a design in the learning data 230 (see Fig. 11) closest to the design of the second object acquired by a data acquisition unit with respect to the image or the feature amount of the image. Further, a second object purchase expectation degree output unit provided in the design evaluation device may calculate the purchase expectation degree from the design of the second object using the purchase expectation degree learning model 240.

In addition, the design evaluation device may further calculate the market share or determine the marketability as in the first embodiment and the modification examples thereof. Specifically, the design evaluation device may output the market share and the marketability related to the most similar design, for example, as the market share and the marketability of the design as the evaluation target. In addition, the design evaluation device may calculate the purchase expectation degree (purchase probability) with respect to a plurality of designs to predict the purchase amount, and output and propose a design having the largest purchase amount as an optimal design for the product.

As another modification example, the design evaluation device may include the second object purchase expectation degree output unit that calculates the purchase expectation degree of the evaluator by using the purchase expectation degree learning model 240 from the design of the object (product) acquired by the design acquisition unit 119 illustrated in Fig. 8, the sensitivity information with respect to the design of the evaluator, and the attribute information of the evaluator. In addition, as in the first embodiment and the modification examples thereof, the design evaluation device may calculate the market share or determine the marketability.

Besides, the design evaluation device may calculate the purchase expectation degree (purchase probability) with respect to a plurality of designs to predict the purchase amount, and output and propose a design having the largest purchase amount as an optimal design for the object. Furthermore, the image feature amount calculation unit 120 and the extraction unit 121 illustrated in Fig. 8 may be provided, and a design in the learning data 230 (see Fig. 11) having a feature amount closest to the feature amount of the design of the object may be specified.

### <<Other Modification Examples>>

Although several embodiments of the present invention have been described above, these embodiments are merely examples and do not limit the technical scope of the present invention. The present invention can take various other embodiments, and various changes such as omissions and substitutions can be made without departing from the gist of the present invention. These embodiments and modifications thereof are included in the scope and gist of the invention described in the present description and the like, and are included in the invention described in the claims and the equivalent scope thereof.

### Reference Signs List

- 100, 100A, 100B: Design evaluation device

- 111: Sensitivity information acquisition unit
- 112: Attribute information acquisition unit
- 113: Similar type classification unit
- 114, 114B: Purchase expectation degree correction value calculation unit
- 115: Purchase expectation degree prediction unit
- 116: Share prediction unit
- 117: Marketability determination unit
- 118: Proposal unit
- 119: Design acquisition unit (data acquisition unit)
- 120: Image feature amount calculation unit
- 121: Extraction unit
- 131: Program
- 140, 140A: Evaluation database
- 160: Correction value database
- 180: Input-output unit
- 200: Learning device
- 210: Purchase behavior acquisition unit
- 220: Learning processing unit (purchase expectation degree learning unit)
- 230: Learning data
- 240: Purchase expectation degree learning model

## Claims

1. A design evaluation device, comprising:
a sensitivity information acquisition unit that acquires sensitivity information of an evaluator with respect to a design of an object;
an attribute information acquisition unit that acquires attribute information of the evaluator; and
a purchase expectation degree prediction unit that predicts a purchase expectation degree with respect to the design of the object on a basis of the sensitivity information and the attribute information.

2. The design evaluation device according to claim 1, further comprising
a share prediction unit that predicts a market share of the object by using the purchase expectation degree.

3. The design evaluation device according to claim 1, further comprising
a marketability determination unit that determines marketability by using the purchase expectation degree and additional information.

4. The design evaluation device according to claim 1, wherein
a plurality of designs for the object is input, and
the design evaluation device further comprises a proposal unit that suggests an optimal design from among the plurality of designs using at least the purchase expectation degree.

5. The design evaluation device according to claim 2, further comprising
a purchase expectation degree correction value calculation unit that calculates a purchase expectation degree correction value for correcting the purchase expectation degree on a basis of the attribute information.

6. The design evaluation device according to claim 1, wherein
the purchase expectation degree is a purchase probability.

7. The design evaluation device according to claim 5, wherein
a market share in the share prediction unit is calculated using the purchase expectation degree that has been corrected with the purchase expectation degree correction value.

8. The design evaluation device according to claim 5, wherein
the purchase expectation degree correction value calculation unit
determines a similar type to which the evaluator belongs from the attribute information, and
calculates a purchase expectation degree correction value allocated to the similar type as a purchase expectation degree correction value for correcting the purchase expectation degree.

9. The design evaluation device according to claim 5, wherein
the purchase expectation degree correction value calculation unit
determines a similar type to which the evaluator belongs from the attribute information, and
calculates a purchase expectation degree correction value assigned to a combination of the similar type and a category of the object as a purchase expectation degree correction value for correcting the purchase expectation degree.

10. The design evaluation device according to claim 1, wherein
the sensitivity information includes at least one of
an answer of the evaluator to an inquiry about a favorable impression of the object,
a time until the answer is made,
an answer of the evaluator to an inquiry about a purchase intention of the object,
a time until the answer is made,
a time during which the evaluator gazes at the design of the object, or
an electroencephalogram measurement result when the evaluator gazes at the design of the object.

11. The design evaluation device according to claim 1, wherein
the sensitivity information acquired by the sensitivity information acquisition unit includes sensitivity information of the evaluator after a price of the object is presented.

12. A learning device, comprising:
a learning data storage unit that stores, as learning data, a set of data of a design of an object, sensitivity information of an evaluator with respect to the design of the object, attribute information of the evaluator, and a purchase expectation degree of the evaluator for the object;
a purchase behavior acquisition unit that acquires purchase behavior information of the evaluator of the object; and
a purchase expectation degree learning unit that associates the purchase behavior information with learning data of the object, and
generates a learned model of the purchase expectation degree with respect to the design.

13. A design evaluation device, comprising:
a data acquisition unit that acquires data of a design of a second object; and
a second object purchase expectation degree output unit that outputs a purchase expectation degree of the second object generated with the data and the learned model generated by the learning device according to claim 12.

14. The design evaluation device according to claim 13, further comprising a share prediction unit that predicts a market share of the second object by using the purchase expectation degree.

15. The design evaluation device according to claim 13 or 14, further comprising
a marketability determination unit that determines marketability by using information of the purchase expectation degree and additional information.

16. The design evaluation device according to any one of claims 13 to 15, wherein
a plurality of designs for an object is input, and
the design evaluation device further comprises a proposal unit that suggests an optimal design from among a plurality of objects using at least the purchase expectation degree.

17. The design evaluation device according to any one of claims 13 to 16, further comprising:
an image feature calculation unit that calculates feature amounts of a design stored in a storage unit of the learning device and a design of the second object; and
an extraction unit that compares the feature amount of the design stored in the storage unit of the learning device with the feature amount of the design of the second object, and extracts a design closest to the design of the second object from among designs in teacher data of the learning device.

18. A program for causing a computer to function as the design evaluation device according to any one of claims 1 to 11 and 13 to 17.

19. A design evaluation method executed by a design evaluation device, the method comprising:
a step of acquiring sensitivity information of an evaluator with respect to a design of an object;
a step of acquiring attribute information of the evaluator; and
a step of predicting a purchase expectation degree with respect to the design of the object on a basis of the sensitivity information and the attribute information.
